(19) Europäisches Patentamt
European Patent Office
Office européen des brevets

(11) **EP 2 669 382 A1**

(12) # EUROPEAN PATENT APPLICATION
published in accordance with Art. 153(4) EPC

(43) Date of publication:
**04.12.2013 Bulletin 2013/49**

(21) Application number: **12739282.7**

(22) Date of filing: **19.01.2012**

(51) Int Cl.:
***C12P 7/06*** *(2006.01)* ***C08B 1/00*** *(2006.01)*
***C12P 7/02*** *(2006.01)* ***C12P 7/16*** *(2006.01)*
***C12P 7/20*** *(2006.01)* ***C12P 7/48*** *(2006.01)*
***C12P 7/54*** *(2006.01)* ***C12P 7/56*** *(2006.01)*
***C12P 19/02*** *(2006.01)* ***C13K 1/02*** *(2006.01)*

(86) International application number:
**PCT/JP2012/051109**

(87) International publication number:
**WO 2012/102175 (02.08.2012 Gazette 2012/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.01.2011 JP 2011015368**

(71) Applicant: **Toyota Jidosha Kabushiki Kaisha**
**Toyota-shi, Aichi 471-8571 (JP)**

(72) Inventors:
- **TABATA Kazuhide**
  **Toyota-shi**
  **Aichi 471-8571 (JP)**
- **ISHIDA Nobuhiro**
  **Nagakute-shi**
  **Aichi 480-1192 (JP)**
- **KATAHIRA Satoshi**
  **Nagakute-shi**
  **Aichi 480-1192 (JP)**
- **NAKAMURA Risa**
  **Nagakute-shi**
  **Aichi 480-1192 (JP)**

(74) Representative: **Albutt, Anthony John**
**D Young & Co LLP**
**120 Holborn**
**London EC1N 2DY (GB)**

(54) **CELLULOSIC BIOMASS TREATMENT METHOD, METHOD OF PRODUCING SUGAR, ALCOHOL OR ORGANIC ACID FROM CELLULOSIC BIOMASS**

(57) This invention is intended to improve saccharification efficiency by allowing cellulose and/or hemicellulose to relax sufficiently with an ionic liquid. Cellulose and/or hemicellulose is allowed to relax sufficiently with an ionic liquid through a step of soaking cellulosic biomass in a solution containing an ionic liquid and an alkali.

Fig. 1

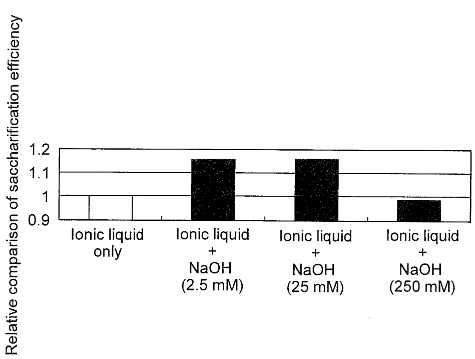


EP 2 669 382 A1

**Description**

Technical Field

The present invention relates to a method of treatment for reduction of the molecular weight of a polysaccharide contained in cellulosic biomass by mixing cellulosic biomass with an ionic liquid and a method for producing a sugar, alcohol, or organic acid by such treatment method.

Background Art

Cellulosic biomass is mainly composed of cellulose, hemicellulose, and lignin. In particular, a polymer of glucose or xylose, such as cellulose or hemicellulose, is a recyclable carbohydrate resource, an alcohol or organic acid represented by ethanol or lactic acid can be produced therefrom, and such resource has thus been drawing attention as an alternative petroleum resource.

In order to produce an alcohol or organic acid from cellulosic biomass, cellulose or hemicellulose contained in cellulosic biomass is hydrolyzed (saccharified) into a constitutive monosaccharide, and the monosaccharide is converted into an alcohol or organic acid via fermentation. As a method for hydrolyzing (saccharifying) cellulose or hemicellulose into a constitutive monosaccharide, methods involving the use of an ionic liquid as described in Patent Documents 1 and 2 have been drawing attention in recent years. By subjecting cellulosic biomass to such a technique, the molecular weight of a polysaccharide, such as cellulose or hemicellulose, contained in cellulosic biomass can be reduced.

In particular, Patent Document 3 discloses a method of treating a lignocellulose-containing starting material with a liquid-treatment medium containing an ionic liquid, and enzymatically hydrolyzing the treated material so as to saccharify the starting material. In addition, Patent Document 4 discloses a technique involving the addition of at least 1 type of delignification catalyst selected from the group consisting of an inorganic acid, an organic acid, an alkali, an oxidant, and the like to a treatment mixture containing an ionic liquid, when soaking lignocellulosic biomass in such treatment mixture.

Prior Art Documents

Patent Documents

Patent Document 1: JP Patent Publication (Kohyo) No. 2005-506401 A
Patent Document 2: JP Patent Publication (Kokai) No. 2009-79220 A
Patent Document 3: WO 2008-090156
Patent Document 4: JP Patent Publication (Kokai) No. 2010-084104 A

Summary of the Invention

Objects to Be Attained by the Invention

Processes for treatment of cellulosic biomass with ionic liquids have suffered from the problem of cellulose and/or hemicellulose not being sufficiently allowed to relax by an ionic liquid, and it was impossible to improve final saccharification efficiency. Accordingly, it is an object of the present invention to provide a method for treatment of cellulosic biomass that sufficiently allows cellulose and/or hemicellulose to relax with the aid of an ionic liquid, and it is another object to provide a method for producing a sugar, alcohol, or organic acid from cellulosic biomass that yields excellent saccharification efficiency by such method for treatment.

Means for Attaining the Objects

The present inventors have conducted concentrated studies in order to attain the above objects. As a result, they discovered that, when mixing cellulosic biomass with an ionic liquid, the addition of an alkali would sufficiently allow cellulose and/or hemicellulose contained in cellulosic biomass to relax, and this would lead to improvement in saccharification efficiency in the subsequent process of saccharification. This has led to the completion of the present invention.

The present invention includes the following.

The method for treatment of cellulosic biomass according to the present invention comprises a step of soaking cellulosic biomass in a solution containing an ionic liquid and an alkali.

The method for production of a sugar according to the present invention comprises: a step of relaxation comprising soaking cellulosic biomass in a solution containing an ionic liquid and an alkali; a step of solid-liquid separation comprising separating the liquid component from the solid component obtained in the step of relaxation; and a step of saccharification of the solid component separated in the step of solid-liquid separation through enzymatic treatment.

The method for production of an alcohol or organic acid according to the present invention comprises: a step of relaxation comprising soaking cellulosic biomass in a solution containing an ionic liquid and an alkali; a step of solid-liquid separation comprising separating the liquid component from the solid component obtained in the step of relaxation; a step of saccharification of the solid component separated in the step of solid-liquid separation through enzymatic treatment; and a step of fermentation of the sugar component obtained in the step of saccharification.

According to the present invention, the solution may contain, in addition to an ionic liquid, an imidazolium salt having a melting point of 100 degrees C or higher.

This description includes part or all of the content as disclosed in the description and/or drawings of Japanese Patent Application No. 2011-015368, which is a priority document of the present application.

Effects of the Invention

According to the present invention, cellulose and/or hemicellulose contained in cellulosic biomass is allowed to relax sufficiently. This can remarkably improve the saccharification efficiency of such cellulose and/or hemicellulose contained in biomass. According to the present invention, therefore, a sugar or organic acid can be efficiently produced with the use of cellulosic biomass.

Brief Description of the Drawings

Fig. 1 is a characteristic diagram showing the results of experiment conducted in Example 1.
Fig. 2 is a characteristic diagram showing the results of experiment conducted in Example 2.

Embodiments for Carrying out the Invention

Hereafter, the method for treatment of cellulosic biomass according to the present invention and the method for production of a sugar, alcohol, or organic acid by such method for treatment are described in detail.

In the present invention, cellulosic biomass is mixed into a solution containing an ionic liquid and an alkali.

<Cellulosic biomass>

According to the present invention, the term "cellulosic biomass" refers to biomass comprising a complex of the cellulose fiber with a crystalline structure, a hemicellulose and lignin. In particular, the cellulose fiber with a crystalline structure and hemicellulose are treated as polysaccharides contained in the cellulosic biomass. Examples of cellulosic biomass include wastes, such as timber from forest thinning, construction and demolition debris, industrial wastes, household wastes, agricultural wastes, wood wastes, forest land remainder materials, and used paper. Examples of cellulosic biomass also include cardboard, used paper, old newspaper, magazines, pulp, and pulp sludge. Further examples of cellulosic biomass include pellets obtained by crushing, compression, and shaping of wood wastes, such as sawdust and shaving, forest land remainder materials, and used paper. Cellulosic biomass may be used in any form. Since relaxation of polysaccharides, such as cellulose and/or hemicellulose contained in cellulosic biomass, is accelerated depending on the ease with which an ionic liquid reacts with cellulosic biomass, it is preferable that cellulosic biomass be finely ground.

<Alkali>

The term "alkali" is a generic term used to refer to any substance that dissolves in water and exhibits the properties of a base. Specifically, alkalis are not particularly limited in the present invention, and examples thereof include hydroxides (salts) of alkali metals and alkaline earth metals. Examples of an alkali contained in the mixture include an alkaline compound containing potassium or sodium, such as KOH, $K_2CO_3$, and NaOH, $Na_2CO_3$, and ammonia.

When alkali concentration is low in the solution, the saccharification efficiency of cellulosic biomass may not be sufficiently improved. In contrast, cellulosic biomass may be excessively degraded when alkali concentration is excessively high in the solution. If cellulosic biomass is treated with the solution and subjected to saccharification with the use of an enzyme, activity of the enzyme used for saccharification may be inhibited because of the excessively high

alkali concentration in the solution, and the saccharification efficiency may not be improved.

<Ionic liquid>

Ionic liquids that can be used for reduction of the molecular weight of cellulosic biomass are not particularly limited. For example, an imidazolium-based ionic liquid, a pyridine-based ionic liquid, an alicyclic amine-based ionic liquid, and an aliphatic amine-based ionic liquid can be used. A compound used as such ionic liquid can be adequately selected in accordance with the degree of molecular weight reduction of cellulose and/or hemicellulose contained in cellulosic biomass. From the viewpoint of the degree of molecular weight reduction of cellulose and/or hemicellulose, use of imidazolium-based ionic liquid composed of an imidazolium compound is preferable. In particular, use of 1,3-dialkyl imidazolium salt as an imidazolium compound is more preferable. Among various types of 1,3-dialkyl imidazolium salts, use of 1-ethyl-3-methylimidazolium chloride is the most preferable.

Examples of imidazolium compounds include 1,3-dialkyl imidazolium salts and 1,2,3-trialkylimidazolium salts. Specific examples of 1,3-dialkyl imidazolium salts include 1-ethyl-3-methylimidazolium bromide, 1-ethyl-3-methylimidazolium chloride, 1-ethyl-3-methylimidazolium (L)-lactate, 1-ethyl-3-methylimidazolium hexafluorophosphate, 1-ethyl-3-methylimidazolium tetrafluoroborate, 1-butyl-3-methylimidazolium chloride, 1-butyl-3-methylimidazolium hexafluorophosphate, 1-butyl-3-methylimidazolium tetrafluoroborate, 1-butyl-3-methylimidazolium trifluoromethanesulfonate, 1-butyl-3-methylimidazolium (L)-lactate, 1-hexyl-3-methylimidazolium bromide, 1-hexyl-3-methylimidazolium chloride, 1-hexyl-3-methylimidazolium hexafluorophosphate, 1-hexyl-3-methylimidazolium tetrafluoroborate, 1-hexyl-3-methylimidazolium trifluoromethanesulfonate, 1-octyl-3-methylimidazolium chloride, 1-octyl-3-methylimidazolium hexafluorophosphate, 1-decyl-3-methylimidazolium chloride, 1-dodecyl-3-methylimidazolium chloride, 1-tetradecyl-3-methylimidazolium chloride, 1-hexadecyl-3-methylimidazolium chloride, and 1-octadecyl-3-methylimidazolium chloride. Examples of 1,2,3-trialkylimidazolium salts include 1-ethyl-2,3-dimethylimidazolium bromide, 1-ethyl-2,3-dimethylimidazolium chloride, 1-butyl-2,3-dimethylimidazolium bromide, 1-butyl-2,3-dimethylimidazolium chloride, 1-butyl-2,3-dimethylimidazolium tetrafluoroborate, 1-butyl-2,3 -dimethylimidazolium trifluoromethanesulfonate, 1-hexyl-2,3-dimethylimidazolium bromide, 1-hexyl-2,3-dimethylimidazolium chloride, 1-hexyl-2,3-dimethylimidazolium tetrafluoroborate, and 1-hexyl-2,3-dimethylimidazolium trifluoromethanesulfonate.

Examples of pyridinium-based ionic liquids include ethylpyridinium salts, butylpyridinium salts, and hexylpyridinium salts. Specific examples of ethylpyridinium salts include 1-ethylpyridinium bromide and 1-ethylpyridinium chloride. Examples of butylpyridinium salts include 1-butylpyridinium bromide, 1-butylpyridinium chloride, 1-butylpyridinium hexafluorophosphate, 1-butylpyridinium tetrafluoroborate, and 1-butylpyridinium trifluoromethanesulfonate. Examples of hexylpyridinium salts include 1-hexylpyridinium bromide, 1-hexylpyridinium chloride, 1-hexylpyridinium hexafluorophosphate, 1-hexylpyridinium tetrafluoroborate, and 1-hexylpyridinium trifluoromethanesulfonate.

Examples of alicyclic amine-based ionic liquids include N,N,N-trimethyl-N-propylammonium bis(trifluoromethanesulfonyl)imide, N-methyl-N-propylpiperidinium bis(trifluoromethanesulfonyl)imide, N,N-diethyl-N-methyl-N-(2-methoxyethyl)ammonium bis(trifluoromethanesulfonyl)imide, and N,N-diethyl-N-methyl-N-(2-methoxyethyl)ammonium tetrafluoroborate.

As described above, anions of an imidazolium-based ionic liquid, a pyridine-based ionic liquid, an alicyclic amine-based ionic liquid, and an aliphatic amine-based ionic liquid may be inorganic or organic anions. Examples of inorganic anions include $Cl^-$, $Br^-$, $I^-$, $NO_3^-$, $BF_4^-$, $PF_6^-$, and $AlCl_4^-$. Examples of organic anions include $CH_3SO_3^-$, $CH_3CH(OH)COO^-$, lactic acid ions, $CH_3COO^-$, $CH_3OSO_3^-$, $CF_3SO_3^-$, $(CF_3SO_2)_2N^-$, and $(C_2F_5SO_2)_2N^-$. Use of an ionic liquid containing $Cl^-$ or $CH_3COO^-$ as an anion is particularly preferable because of the rapid rate at which it dissolves the cellulose and/or hemicellulose contained in cellulosic biomass.

<Imidazolium salt having melting point of 100 degrees C or higher>

A solution containing an ionic liquid and an alkali may contain "an imidazolium salt having a melting point of 100 degrees C or higher," in addition to the ionic liquid described above. The term "an imidazolium salt having a melting point of 100 degrees C or higher" refers to a salt composed of a cation having an imidazole ring and an anion, and having a melting point of 100 degrees C or higher, which is not an ionic liquid.

A cation constituting an imidazolium salt having a melting point of 100 degrees C or higher can be represented by the following formula:

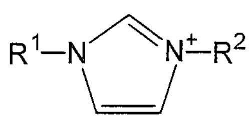

wherein $R^1$ and $R^2$ are each independently selected from the group consisting of: $C_{1-10}$ alkyl, such as methyl, ethyl, n-propyl, isopropyl, n-butyl, sec-butyl, tert-butyl, pentyl, hexyl, heptyl, octyl, nonyl, and decyl; substituted or unsubstituted $C_{3-10}$ cycloalkyl, such as cyclopropyl, methylcyclopropyl, cyclohexyl, 2,6-dimethylcyclohexyl, 2,6-diethylcyclohexyl, 2,4,6-trimethylcyclohexyl, 2,4,6-triethylcyclohexyl, and cyclodecyl; $C_{2-10}$ alkenyl, such as allyl; and aromatic hydrocarbon, such as phenyl, 2,6-dimethylphenyl, 2,6-diisopropylphenyl, 2,4,6-trimethylphenyl, tolyl, and naphthyl.

Preferably, $R^1$ and $R^2$ are each independently selected from the group consisting of: $C_{1-6}$ alkyl (e.g., methyl, ethyl, n-propyl, isopropyl, n-butyl, sec-butyl, tert-butyl, pentyl, and hexyl); substituted or unsubstituted cyclohexyl (e.g., cyclohexyl, 2,6-dimethylcyclohexyl, and 2,4,6-trimethylcyclohexyl); allyl; and substituted or unsubstituted phenyl (e.g., phenyl, 2,6-dimethylphenyl, 2,6-diisopropylphenyl, and 2,4,6-trimethylphenyl). It is more preferable that $R^1$ and $R^2$ each represent the same substituent.

An anion constituting an imidazolium salt having a melting point of 100 degrees C or higher may be an inorganic or organic anion. Examples of inorganic anions include $Cl^-$, $Br^-$, $I^-$, $NO_3^-$, $BF_4^-$, $PF_6^-$, and $AlCl_4^-$. Examples of organic anions include acetic acid anion, phosphoric acid anion, lactic acid anion, methanesulfonic acid anion, trifluoromethanesulfonic acid anion, bis(trifluoromethanesulfonyl)imide anion, and bis(pentafluoroethanesulfonyl)imide anion. Preferably, an anion constituting an imidazolium salt having a melting point of 100 degrees C or higher is selected from the group consisting of $Cl^-$, $Br^-$, $I^-$, acetic acid anion, and phosphoric acid anion.

Specific examples of imidazolium salts having a melting point of 100 degrees C or higher that are preferable in the present invention include 1,3-dimethylimidazolium chloride, 1,3-dimethylimidazolium dimethyl phosphate, 1,3-dicyclohexyl imidazolium chloride, 1,3-bis(2,4,6-trimethylphenyl)imidazolium chloride, and 1,3-bis(2,6-diisopropylphenylethyl)imidazolium chloride.

<Step of treatment>

Treatment of cellulosic biomass with the solution described above may involve soaking of the cellulosic biomass in the solution mentioned above. Alternatively, the cellulosic biomass may be soaked in such solution and subjected to agitation, ultrasonic application, or vortex mixing in such state, according to need.

According to the present invention, the temperature at which cellulosic biomass is treated with the solution mentioned above is not particularly limited. Treatment is preferably carried out at 60 degrees C to 150 degrees C, and most preferably at 80 degrees C to 120 degrees C. When the temperature is lower than 60 degrees C, the saccharification efficiency may not be sufficiently improved. In contrast, cellulosic biomass may be excessively degraded, and the treatment cost may increase, when the temperature is higher than 150 degrees C.

As described above, the treatment method of the present invention can be performed as a previous step to the step of saccharification through enzymatic treatment of a polysaccharide contained in cellulosic biomass. Specifically, cellulosic biomass is allowed to relax through the treatment method of the present invention, and it becomes easy to proceed with the subsequent step of saccharification. As a result, the saccharification efficiency of cellulosic biomass would be remarkably enhanced and improved through the treatment method of the present invention. After the treatment method described above, conventional techniques, such as filtration or centrifugation, may be carried out to separate cellulosic biomass from the solution.

<Step of saccharification>

In the step of saccharification, cellulose is hydrolyzed into a monosaccharide such as glucose through saccharification via enzymatic treatment, and hemicellulose is hydrolyzed into a monosaccharide such as xylose, arabinose, or mannose. An enzyme known in the art, such as cellulase or hemicellulase (e.g., xylase, arabinose, or mannase), capable of hydrolyzing cellulose or hemicellulose is adequately used for enzymatic treatment. A chemically synthesized enzyme, a mixture of purified microbial products, or a mixture of microorganisms synthesizing a target enzyme may be used.

A cellulase used in the present invention is preferably selected from the group consisting of a cellulase derived from a species in the genus *Trichoderma* (*Trichoderma reesei* or *Trichoderma viride,* in particular), a cellulase derived from a species in the genus *Aspergillus* (*Aspergillus niger,* in particular), a cellulase derived from a species in the genus *Pyrococcus* (*Pyrococcus horikoshii,* in particular), a cellulase derived from a species in the genus *Humicola* (*Humicola insolens,* in particular), a cellulase derived from a species in the genus *Phanerochaete* (*Phanerochaete chrysosporium,* in particular), and a mixture of two or more thereof.

Cellulosic biomass treated with the solution of the present invention is more likely to be saccharified by cellulase because cellulose or hemicellulose is allowed to relax sufficiently with a solution containing an alkali and ionic liquid. Thus, the higher saccharification efficiency can be attained, compared with a case in which cellulase is allowed to react with cellulosic biomass by soaking cellulosic biomass in a solution consisting of an ionic liquid.

<Step of production of alcohol and/or organic acid>

An alcohol or organic acid can be produced by so-called fermentation that makes use of a sugar component obtained in the step of saccharification. For example, ethanol, propanol, butanol, or glycerine can be produced as an alcohol, and lactic acid, acetic acid, citric acid, oxalic acid, succinic acid, β-hydroxybutyric acid, or 3-hydroxypropionic acid can be produced as an organic acid.

Any microorganisms can be used for fermentation without particular limitation, provided that a target product can be produced with the use of a sugar component, such as a monosaccharide or oligosaccharide, obtained in the step of saccharification. When ethanol is to be produced, for example, *Saccharoniyces cerevisiae* or *Schizosaccharomyces pombe* can be employed. Alternatively, bacteria, such as *E. coli,* into which genes necessary for ethanol biosynthesis with the use of a monosaccharide or oligosaccharide substrate have been introduced, may be used when ethanol is to be produced. When lactic acid is to be produced, for example, conventional lactic acid-producing bacteria, such as bacteria of the genus *Lactobacillus,* can be employed. In addition, *E. coli* cells or yeast cells into which genes necessary for lactic acid biosynthesis with the use of a monosaccharide or oligosaccharide substrate have been introduced may also be used.

In the method for production of an alcohol or organic acid according to the present invention, the step of saccharification and the step of fermentation may be carried out in separate tanks, and the saccharification product may be transferred to a fermentation tank so as to perform the step of fermentation. Alternatively, the step of saccharification and the step of fermentation may be simultaneously carried out in the same tank.

After the completion of the step of fermentation, a target product such as an alcohol or organic acid can be recovered and produced in accordance with a conventional technique. When ethanol is to be produced, for example, distillation or pervaporation can be performed.

Examples

Hereafter, the present invention is described in greater detail with reference to the examples, although the technical scope of the present invention is not limited to the following examples.

[Example 1]

In this example, 1-butyl-3-methylimidazolium acetate (hereafter referred to as "[Bmim][Ac]," Solbionic) was used as ionic liquid. The structural formula representing such ionic liquid is shown below.

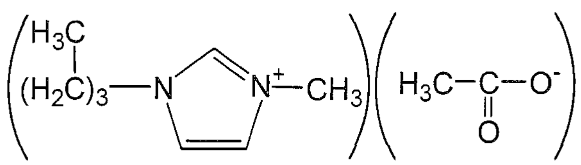

[Bmim] [Ac] (1.0 g) was collected in a vial, and sodium hydroxide was added thereto so as to prepare a solution used for soaking cellulosic biomass therein. In this example, solutions having sodium hydroxide concentration of 2.5 mM, 25 mM, and 250 mM were prepared.

<Treatment of cellulosic biomass>

In this example, eucalyptus powder crushed with the use of a cutter mill (average particle diameter: 150 mm) was used as the cellulosic biomass. In this example, 50 mg of eucalyptus powder was added to the solution prepared in the manner described above. Thereafter, cellulosic biomass was soaked in the solution and allowed to stand therein at 120 degrees C for 30 minutes. The resultant was then washed with 9 ml of sterilized water and, with the use of a filter, cellulosic biomass was washed several times with sterilized water to wash away the treatment solution.

<Saccharification reaction using cellulase>

After the treatment, cellulosic biomass washed with sterilized water was collected in a vial again, 9.9 ml of 10 mM citrate buffer (pH 5.5) was added thereto, and 0.1 ml of a cellulase mixture was then added thereto. The resulting sample was maintained at 40 degrees C and subjected to the saccharification reaction. The reaction product was sampled 72 hours later and the glucose concentration in the solution was measured.

Novozyme Celluclast originating from *Trichoderma reesei* ATCC 26921 (Sigma-Aldrich) was mixed with Novozyme 188 originating from *Aspergillus niger* (Sigma-Aldrich) at a proportion of 5: 1, the resulting mixture was adjusted to 6 FPU/g biomass, and the resultant was used as the cellulase mixture. Glucose concentration was measured using a Biosensor BF-5 (Oji Scientific Instruments) in accordance with the protocols attached thereto.

Based on the determined glucose concentration (%), the efficiency of conversion into sugar was determined in accordance with the formula shown below by designating the number of glucose units in cellulose contained in each biomass sample as 100. The number of glucose units in cellulose was determined based on the results of component analysis of the biomass sample.

$$\text{Glucose conversion efficiency (\%)} = \frac{\text{amount of glucose produced}}{\text{number of glucose units in biomass}} \times 100$$

Fig. 1 shows the determined glucose conversion efficiency. As is apparent from Fig. 1, the glucose conversion efficiency after the saccharification treatment was significantly improved with the addition of an alkali of given concentration to ionic liquid. In this example, sodium hydroxide is used as an alkali. In such a case, glucose conversion efficiency is improved when sodium hydroxide concentration is 2.5 mM or 25 mM.

[Example 2]

In this example, cellulosic biomass was treated in the same manner as in Example 1, except that 1 mg of 1,3-bis(2,6-diisopropylphenyl)imidazolium chloride was added as "an imidazolium salt having a melting point of 100 degrees C or higher" to the solution used for soaking cellulosic biomass therein. Thereafter, the resultant was subjected to saccharification. The structural formula of 1,3-bis(2,6-diisopropylphenyl)imidazolium chloride is shown below.

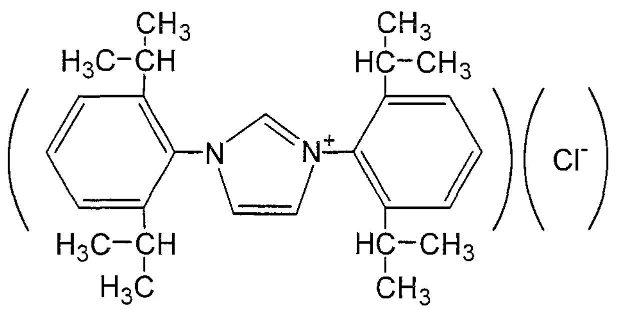

Fig. 2 shows the determined glucose conversion efficiency. In comparison with the results of Example 1 shown in Fig. 1, the glucose conversion efficiency after saccharification is further improved with the addition of "an imidazolium salt having a melting point of 100 degrees C or higher," in addition to an alkali of given concentration to ionic liquid.

All publications, patents, and patent applications cited herein are incorporated herein by reference in their entirety.

## Claims

1. A method for treatment of cellulosic biomass comprising a step of soaking cellulosic biomass in a solution containing an ionic liquid and an alkali.

2. The method for treatment of cellulosic biomass according to claim 1, wherein the solution further contains an imidazolium salt having a melting point of 100 degrees C or higher.

3. A method for production of a sugar from cellulosic biomass comprising a step of soaking cellulosic biomass in a solution containing an ionic liquid and an alkali by the method according to claim 1 or 2 and a step of allowing cellulase to react with cellulosic biomass to produce a sugar.

4. A method for production of an alcohol or organic acid comprising a step of soaking cellulosic biomass in a solution

containing an ionic liquid and an alkali by the method according to claim 1 or 2, a step of allowing cellulase to react with the treated cellulosic biomass to produce a sugar, and a step of fermentation of the obtained sugar component to produce an alcohol or organic acid.

**5.** The method for production of an alcohol or organic acid according to claim 4, wherein the step of producing a sugar is simultaneously carried out with the step of producing an alcohol or organic acid.

Fig. 1

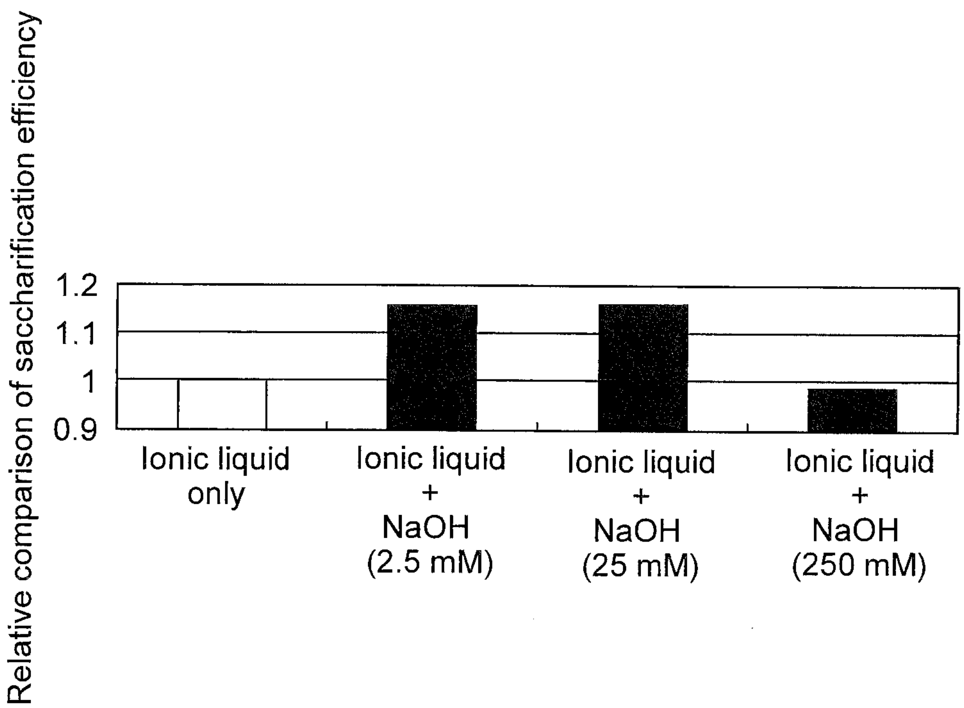
Relative comparison of saccharification efficiency
1.2
1.1
1
0.9
Ionic liquid only
Ionic liquid + NaOH (2.5 mM)
Ionic liquid + NaOH (25 mM)
Ionic liquid + NaOH (250 mM)

Fig. 2

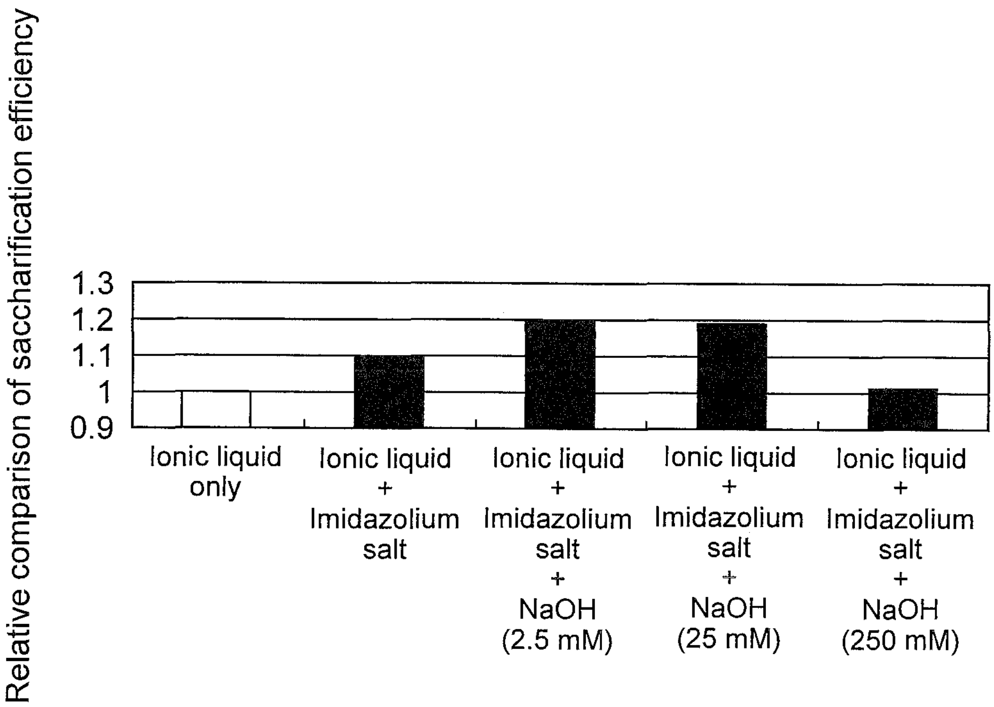
Relative comparison of saccharification efficiency
1.3
1.2
1.1
1
0.9
Ionic liquid only
Ionic liquid + Imidazolium salt
Ionic liquid + Imidazolium salt + NaOH (2.5 mM)
Ionic liquid + Imidazolium salt + NaOH (25 mM)
Ionic liquid + Imidazolium salt + NaOH (250 mM)

**INTERNATIONAL SEARCH REPORT**

International application No.
PCT/JP2012/051109

A. CLASSIFICATION OF SUBJECT MATTER
*C12P7/06*(2006.01)i, *C08B1/00*(2006.01)i, *C12P7/02*(2006.01)i, *C12P7/16*(2006.01)i, *C12P7/20*(2006.01)i, *C12P7/48*(2006.01)i, *C12P7/54*(2006.01)i, *C12P7/56*(2006.01)i, *C12P19/02*(2006.01)i, *C13K1/02*(2006.01)i
According to International Patent Classification (IPC) or to both national classification and IPC

B. FIELDS SEARCHED
Minimum documentation searched (classification system followed by classification symbols)
C12P7/06, C08B1/00, C12P7/02, C12P7/16, C12P7/20, C12P7/48, C12P7/54, C12P7/56, C12P19/02, C13K1/02

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus/REGISTRY(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | US 2007/0161095 A1 (Michael H. Gurin),<br>12 July 2007 (12.07.2007),<br>claims 1, 2; paragraph [0023]<br>& EP 2002010 A & WO 2007/112090 A2 | 1<br>1,3-5<br>2 |
| X<br>A | WO 2010/012132 A1 (CHINA FUEL (HUAIBEI) BIOENERGY TECHNOLOGY DEVELOPMENT CO., LTD.),<br>04 February 2010 (04.02.2010),<br>claims 1, 5, 6; example 5<br>& EP 2322588 A1 & JP 2011-529091 A | 1<br>2 |
| X<br>A | US 2010/0279372 A1 (Hwa Young CHO),<br>04 November 2010 (04.11.2010),<br>claims 1 to 3; paragraphs [0056], [0091] to [0092]<br>& KR 10-2010-0119018 A | 1,3-5<br>2 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 February, 2012 (24.02.12) | 06 March, 2012 (06.03.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/051109

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | JP 2009-189277 A (Toyota Motor Corp.),<br>27 August 2009 (27.08.2009),<br>claims 1, 13, 14<br>(Family: none) | 1,3-5<br>2 |
| Y<br>A | JP 2010-521155 A (The University of Toledo),<br>24 June 2010 (24.06.2010),<br>claim 1<br>& US 2008/0227162 A1 & EP 2137318 A<br>& WO 2008/112291 A2 & CN 101765663 A | 1,3-5<br>2 |
| Y<br>A | JP 2008-274247 A (National Institute of Advanced Industrial Science and Technology),<br>13 November 2008 (13.11.2008),<br>claims 3, 16; paragraphs [0053], [0068], [0085]<br>& US 2010/0151527 A1 & EP 2133366 A1<br>& WO 2008/123419 A1 | 1,3-5<br>2 |
| Y | WO 2009/059234 A2 (NOVOZYMES, INC.),<br>07 May 2009 (07.05.2009),<br>page 16, lines 24 to 31<br>& US 2009/0130707 A1 & EP 2235191 A<br>& CN 101910406 A | 1,3-5 |
| P,Y | JP 2011-254727 A (Toyota Motor Corp.),<br>22 December 2011 (22.12.2011),<br>claim 1<br>(Family: none) | 2 |

Form PCT/ISA/210 (continuation of second sheet) (July 2009)

**INTERNATIONAL SEARCH REPORT**

International application No.
PCT/JP2012/051109

Examination is made on claim 2 and claims 3-5 which refer to claim 2. These claims are defined by a melting point, for example, in such a manner "an imidazolium salt having a melting point of 100°C or higher". Therefore, these claims include all of imidazolium salts having the above-stated property. However, it is generally difficult to analogize the melting points of all of imidazolium salts from the chemical structures thereof. Therefore, those imidazolium salts which are disclosed in the meaning within PCT Article 5 are only a few examples, and therefore it cannot be considered that these inventions are supported by the disclosure of the description in the meaning within PCT Article 6.

Such being the case, the search was carried out on the imidazolium salts which are described particularly in paragraph [0031] of the description.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005506401 A
- JP 2009079220 A
- WO 2008090156 A
- JP 2010084104 A
- JP 2011015368 A